# EUROPEAN PATENT APPLICATION

(11) **EP 4 011 269 A1**
(43) Date of publication of application: **15.06.2022**
(21) Application number: 20212622.3
(22) Date of filing: 09.12.2020
(51) Int. Cl.: A61B 1/005, A61B 1/008, A61B 1/00

(54) **ARTICULATING SHAFT FOR A STEERABLE CATHETER SYSTEM, CATHETER, AND FABRICATION METHOD**

(71) Applicant: Creganna Unlimited Company, Ballybrit, Galway H91 VN2T (IE)
(72) Inventor: MC DERMOTT, Bernard, Claremorris F12 PD69 (IE); SZCZEPANSKI, Adam, Limerick (IE); PHELAN, Richard, Piltown Kilkenny (IE); MULDOON, Damian, Loughrea GW H62 KPO3 (IE)
(74) Representative: Grünecker Patent- und Rechtsanwälte PartG mbB

(57) **Abstract**

The present invention relates to an articulating shaft for a steerable catheter system suitable for use in endoscopic procedures or in minimally invasive procedures. The present invention also relates to a steerable catheter system comprising such an articulating shaft and to a method of fabricating such a shaft. The shaft (100) comprises a tubular body (101) with a longitudinal central axis (118), the tubular body having at least one main lumen (104) with a distal end and a proximal end, and at least one wire support element (106) forming at least one wire lumen (108) for supporting at least one actuating wire (128). The main lumen (104) and the wire lumen (108) are integrally formed in an extrusion part having a uniform hardness in an axial and a radial direction, wherein the shaft (100) further comprises a plurality of removed sections or cuts (110) which extend in a radial direction and are distanced from each other in an axial direction.

## Description

The present invention relates to a low cost articulating shaft for a steerable catheter system suitable for use in endoscopic procedures or in minimally invasive procedures. The present invention also relates to a steerable catheter system comprising such an articulating shaft and to a method of fabricating such a shaft.

Endoscopes are used to examine internal organs of the body, inserted through small incisions or through natural orifices. They are typically long slender and flexible sheaths with a camera or fiber optic bundle and lens system to relay images to the user, and a lighting system to illuminate the internal structures. They typically have a steering system to deflect the tip in tight bend radius either in one, two or all directions to allow the user to position the tip where required. Endoscopes generally have other working lumens depending on their type: For instance, one or more lumens are provided for the passage of biopsy and therapeutic instruments, for irrigation of the tip to clean the lenses, for aspiration of bodily fluids and for passage of air to insufflate body cavities to create space.

Therapeutic endoscopy is an endoscopic procedure during which treatment is carried out via the endoscope. Diagnostic endoscopy is used to visualize a part of the gastrointestinal, respiratory, or urinary tract in order to aid diagnosis.

Endoscopes are generally reusable and are designed to perform a high number of procedures and survive a sterilization process after each procedure. This results in a high acquisition cost and high costs associated with disinfection, maintenance, and repair.

A low acquisition cost is required for single use or disposable devices. Such devices have no sterilization and maintenance costs and have no risk of cross contamination.

Known endoscopes are generally constructed from a large number of components, often with distal end articulation sections comprised of complex multi-segment hinged metal or polymer components with additional components to align and contain the required pull wires. This articulating section is generally attached to a multilayered proximal section sheath constructed from some or all of the following elements: braids, coils, liners, polymer jackets, multi lumen extrusion cores, pull wires and pull wire liners.

This type of construction allows high flexibility to enable articulation in the distal section with lower flexibility and greater pushability in the proximal section, along with a long service life.

Other conventional construction methods may be based on a single piece steel tube design with laser cut features along the length, changing the flexibility and pushability properties of the tube from the distal section to the proximal section.

Further known constructions comprise a braided sheath containing lumens built into the wall of the sheath to contain and align the pull wires used for articulation. These shafts generally have a single large internal lumen and this can be subdivided further by means of a multi lumen extrusion or by a single lumen working channel tube inserted through the braided sheath main lumen. These types of constructions require that the flexibility be changed from the highly flexible articulating distal end of the shaft to the less flexible and pushable proximal section. This is achieved mainly by using multiple durometer polymer materials in the construction of the braided shaft, with low durometer flexible polymer matrix used in the distal section and high durometer, less flexible and more pushable material used for construction of the proximal section and typically a material with a durometer between that of the distal and proximal section to act as a transition section between the soft and the hard sections.

All these conventional construction types require additional components to provide additional lumens to complete the device.

There is still a need for an articulating shaft that has improved mechanical properties, at the same time improving the fabrication process and reducing the costs of production.

The majority of endoscopic devices are required to have a distal section which can be steered in one or more directions with defined and consistent bend geometry. This requires lumens or other features to accurately align the articulation wires or cables used to deflect the distal section of the device to the required bend radius. It further requires additional lumens to align and position the various elements needed for operation of the device, lumens for the camera leads or fiber optic bundle, lumens for the light transmission or power cables and one or more working channel lumens.

Furthermore, the shaft proximal of the articulating section needs to be axially stiff to allow it to be pushed through the anatomy to reach its target area.

This proximal region also needs to have a certain level of flexibility to allow it to track easily through the anatomy to reach its target area.

To be practical for use as a disposable device, the shaft needs to have a low construction and materials cost.

These objectives are solved by the subject matter of the independent claims. Advantageous embodiments of the present invention are the subject matter of the dependent claims.

The present disclosure provides a low cost steerable device that can be constructed from a single durometer multi lumen extrusion (MLE). An MLE consists of an extruded element of typically circular cross section with a number of axially aligned holes or lumens running continuously through and generally within the outline of the section, extending throughout the length of the tube. The sizes and positions of these lumens can be arranged in numerous ways according to the design requirements. Such polymer extrusions are formed into a continuous profile to be subsequently cut to required lengths.

Advantageously, the single durometer MLE can be cut in various ways to modify its flexibility along the length as required, from a relatively stiff and pushable proximal section with non to minimal cutting, to a highly flexible distal steerable section with cuts made to allow articulation in one or more directions.

Moreover, the single MLE provides all the required lumens and features to guide and position the necessary pull wires required for articulation and axial support wires to provide increased axial stiffness and reinforce or provide hinges for the bending of the sections. Additional lumens can be easily provided to guide and position other elements needed in the assembly of devices using such a construction; leads for electronic devices, fiber optic cables and working channels for instance. Additional flexible tubes can be inserted into the extrusion lumens to provide fluid tight or continuous closed lumens used for the passage of various medical instruments and for aspiration, irrigation and insufflation.

Further, a flexible outer jacket can be fitted to the outside of the MLE to protect the inner elements from the external environment. This construction results in a steerable sheath composed of a minimal number of relatively low cost components.

The central and generally larger lumen may also be referred to as the working lumen, main lumen or core lumen. To enable steering of the shaft, at least one steering wire (also called "pull wire" or "actuating wire" in the following) can be inserted into the steering wire lumen that is formed by a plurality of aligned openings which are provided in wire support elements. These wire support elements are separated from each other by removed sections or cuts, so that bending of the shaft is facilitated. The outer wall of the shaft contains such removed sections or cuts to allow the shaft to contract and bend on this side.

In particular, the present invention provides an articulating shaft for a steerable catheter system, the shaft comprising a tubular body with a longitudinal central axis, the tubular body having at least one main lumen with a distal opening and a proximal opening, and a plurality of wire support elements forming a wire lumen for supporting at least one actuating wire. The main lumen and the wire lumen are integrally formed in an extruded part having a uniform material hardness, wherein the shaft further comprises a plurality of removed sections or cuts which extend in a radial direction and are distanced from each other in an axial direction.

The hardness, which may also be referred to as "durometer", is intended to mean the shore D hardness, a parameter specific to the chosen polymer material.

An advantage of this solution can be seen in the fact that the assembly process is facilitated due to the radial one-piece design of the shaft. Furthermore, the articulating shaft has a high degree of pliability, at the same time ensuring a high precision of the steering. In particular, the present disclosure allows the fabrication of an articulating shaft for a steerable catheter system that may be based on a so-called multi lumen extrusion (MLE) part. Such a shaft may be fabricated from a minimal number of relatively low-cost components. The extruded part may for instance be manufactured from a hard polymer material or any other suitable material. Advantageously, the single MLE provides all the required lumens and features to position and align the various elements required for the assembled device in a single part, including lumens for electrical wires and/or cables and fiber optic cables and also one or more working lumens. It also provides the lumens for the articulation system. Furthermore, the shaft may use a single MLE which functions as the distal articulation section and also as the proximal pushable section.

According to a further advantageous example, the shaft includes a flexible outer jacket encompassing the tubular body. Such a jacket (or sheath) may be fabricated from a polymer and provides a smooth surface for contact with anatomy and serves to protect the other components of the completed device from external substances, for instance bodily fluids. The outer jacket may for instance comprise a simple extruded tube or a braid or coil reinforced tube or other construction.

Furthermore, at least one main lumen and/or any of the other lumens may comprise a flexible tube inserted into the tubular body. The tube may for instance be fabricated from a polymer material and provides a fluid tight channel.

According to an advantageous embodiment of the present invention, the removed sections or cuts extend by at least 180° in a radial direction around the body. Thus, a sufficiently large segment of the shaft has enough pliability to facilitate the steering process. For instance, each removed sections or cuts may be provided to extend around approximately half the circumference of the shaft. Each removed sections or cuts may have a gradually diminishing dimension in an axial direction towards their peripheral regions around the circumference of the shaft. This design allows for an optimized force distribution when bending the shaft. The removed sections or cuts can be mechanically cut or laser cut, by means of a gang saw, grinder, blades, or other methods.

According to an advantageous embodiment, the shaft comprises a plurality of discrete segments aligned along the longitudinal central axis. These segments may be aligned and held together by at least one actuating wire and reinforcement wires (also referred to as support wires) like beads on a string. This design has the advantage that the articulating shaft may be composed from a plurality of shorter parts, thus enhancing the flexibility of the manufacturing process. Moreover, the pliability of the articulating shaft is enhanced significantly by facilitating the compression on the inner bending radius of the shaft and, additionally, facilitating the expansion on the outer bending radius of the shaft. The individual segments may have cross-sectional faces which are either essentially orthogonal to the central axis, or at least a part of the cross-sectional face may include an angle with the central longitudinal axis.

Furthermore, the discrete segments may be aligned with separate spacer elements between each pair of adjacent segments. This allows the segments to be formed with a simple geometry, at the same time still providing pliability and accurate steering. For instance, the segments each may have the shape of a straight circular cylinder and the spacer elements have an essentially C-shaped radial outline with a uniform axial thickness, leaving axially distanced openings between the segments. Such openings may be aligned on one side of the shaft when one-way steering is desired. Alternatively, the openings may be arranged on two opposing radial portions of the shaft to facilitate bending in two directions for a two-way steering catheter.

Instead of or in addition to such spacers, each of the segments may have a first and a second lateral face delimiting the segment in an axial direction, wherein the lateral face is at least partly inclined to the longitudinal axis at an angle different from 90°. Such inclined faces may be provided for facilitating bending in one direction or in two directions. For instance, the faces may deviate from the orthogonal direction by about 20°.

According to a further advantageous embodiment, the shaft has a circular radial outline and the removed sections or cuts have a wedge-shaped tapered outline, wherein the wire support elements are located in a region where the removed sections or cuts have their minimum axial dimension. This geometry allows for a particularly precise steering via the actuating wire. For one-way steering, the redundant steering lumen can be used for an additional support wire to enhance torque performance.

Furthermore, in order to further enhance the pliability of the shaft, a plurality of first removed sections or cuts is provided separating a plurality of first wire support elements from each other, wherein a plurality of second removed sections or cuts is provided opposing the first removed sections or cuts in a radial direction and being arranged offset from the plurality of first removed sections or cuts in an axial direction. Thereby, the pliability of the articulating shaft is enhanced significantly by facilitating the compression on the inner bending radius of the shaft and, additionally, facilitating the expansion on the outer bending radius of the shaft.

A symmetric distribution of forces can be achieved when the main lumen is arranged concentrically within the outline of the shaft. Alternatively, if only a one-way steering is intended, the main lumen may be arranged within the outline of the shaft with its central axis being distanced from the longitudinal central axis of the shaft, thereby leaving room in the cross-section of the shaft for additional lumens, for instance for electrical or optical cabling.

A geometry with a maximized lumen cross sectional area can be achieved if at least one wire support element comprises a tube shaped wire lumen with an inner wall and an outer wall, the outer wall being partly formed by an inner wall of the main lumen.

In order to allow the use of the shaft with a two-way steering system, i. e. with two pull wires pulling the shaft in two different, preferably opposing, directions, the shaft may comprise a first and a second row of wire support elements axially extending along the longitudinal axis and having first and second feed-through openings, respectively, the rows being arranged opposite to each other in a radial direction, wherein the first feed-through openings are aligned opposite to the second feed-through openings.

According to an advantageous example, the shaft has a circular radial outline. Such a design is compatible with existing steering systems and allows optimal force distribution.

According to a further advantageous example, the shaft further comprises two axial support lumens for receiving axial support wires. The axial support wires, which are assembled into these lumens, can be made from steel or Nitinol or other suitable materials with some elastic properties.

When the actuating wire is attached to the distal end of the assembly and a tensile load is applied then the wire shortens. This causes the openings formed by the removed sections or cuts to close, causing the assembly to bend in the direction of the openings. The neutral axis support wires, which are also attached at the distal and proximal ends, bend to allow the assembly to bend. The neutral axis support wires also carry some of the articulation axial load. The wires keep the segments in alignment and position, and also keep tension on the assembly, preventing the segments from separating. As mentioned above, for one-way steering, the redundant steering lumen can be used for an additional support wire, unattached at the proximal end and floating in its lumen to enhance the torque performance.

In order to facilitate the bending, the axial support lumen may be at least partly segmented by peripheral regions of the removed sections or cuts.

Furthermore, other lumens, for instance for electric cables or the like may also be provided in the shaft according to the present invention.

The present disclosure further relates to a wire articulated steerable catheter system comprising an articulating shaft according to the present invention. The catheter system further comprises at least one actuating wire passing through the wire lumen, wherein the actuating wire has a proximal end and a distal end, the actuating wire being fixed at the distal end so that pulling the actuating wire at the proximal end effects steering of the catheter, and control means located at a proximal portion of the catheter for controlling the pulling on the proximal end of the actuating wire.

As used herein, the terms "proximal" and "distal" are to be taken as relative to a user using the disclosed delivery devices. "Proximal" is to be understood as relatively closer to the user and "distal" is to be understood as relatively farther away from the user.

Finally, the present invention further relates to a method for fabricating an articulating shaft for a steerable catheter system, the method comprising:
providing a tubular body with a longitudinal central axis, the tubular body having at least one main lumen with a distal opening and a proximal opening, and at least one wire support element forming at least one wire lumen for supporting at least one actuating wire,
wherein the main lumen and the wire lumen are integrally formed in an extrusion part having a uniform hardness in an axial and a radial direction,
providing a plurality of removed sections or cuts which extend in a radial direction and are distanced from each other in an axial direction.

As mentioned above, the assembly process is facilitated due to the radial one-piece design of the shaft. Furthermore, the articulating shaft has a high degree of pliability, at the same time ensuring a high precision of the steering.

In particular, a high freedom of design with low cost can be achieved because the shaft is fabricated by means of an extrusion process. For instance, a possible construction of a two-way steerable device is as follows. A multi lumen extrusion (MLE) is extruded from a hard polymer material. This extrusion contains a number of lumens (holes) which extend through the length of the extrusion. The polymer material may be chosen so that the extrusion properties meet the stiffness/flexibility and pushability properties required for the proximal section of the shaft. Alternatively, the material may be chosen so that the extrusion is stiffer than that required for the proximal section. In this case the proximal section will need to be mechanically modified to increase its flexibility. Numerous polymer materials can be used, including inherently lubricious materials such PTFE.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form-individually or in different combinations-solutions according to the present invention. The following described embodiments thus can be considered either alone or in an arbitrary combination thereof. Further features and advantages will become apparent from the following more particular description of the various examples of the disclosure, as illustrated in the accompanying drawings, in which like references refer to like elements, and wherein:
- **FIG. 1**: is a schematic perspective representation of an articulating shaft according to a first example;
- **FIG. 2**: is a detail of Fig. 1;
- **FIG. 3**: is a further detail of Fig. 1;
- **FIG. 4**: is a schematic perspective representation of a segment of an articulating shaft according to a further example;
- **FIG. 5**: is a schematic perspective representation of an articulating shaft according to another example;
- **FIG. 6**: is a schematic perspective representation of an articulating shaft according to a further example;
- **FIG. 7**: is a schematic perspective representation of an articulating shaft according to a further example;
- **FIG. 8**: is a schematic perspective representation of an articulating shaft according to a further example;
- **FIG. 9**: is a schematic perspective representation of an articulating shaft according to a further example;
- **FIG. 10**: is a schematic perspective representation of an articulating shaft according to a further example;
- **FIG. 11**: is a schematic perspective representation of an articulating shaft according to a further example;
- **FIG. 12**: is a schematic perspective representation of an articulating shaft according to a further example;
- **FIG. 13**: is a schematic perspective representation of an articulating shaft according to a further example;
- **FIG. 14**: is a schematic perspective representation of a multilumen extrusion.

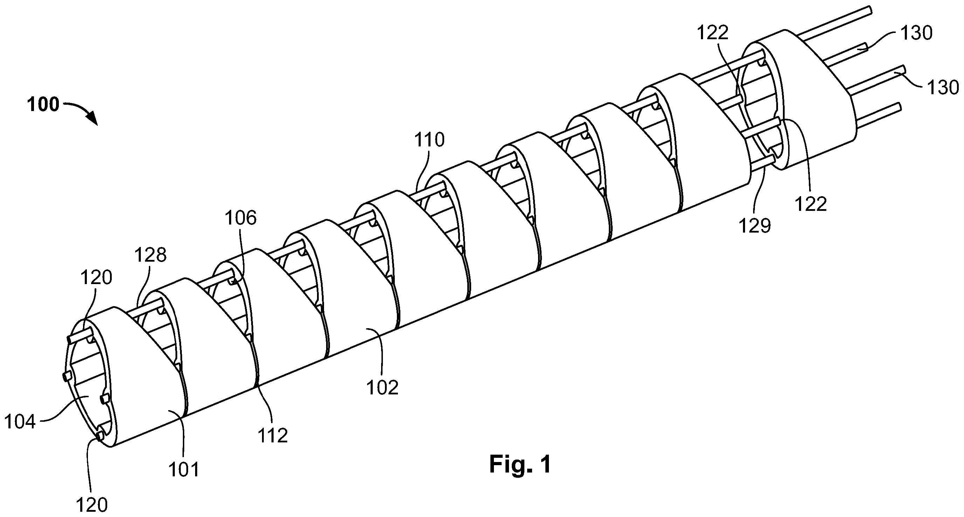

The present invention will now be explained in more detail with reference to the Figures and firstly referring to Fig. 1 to 3.

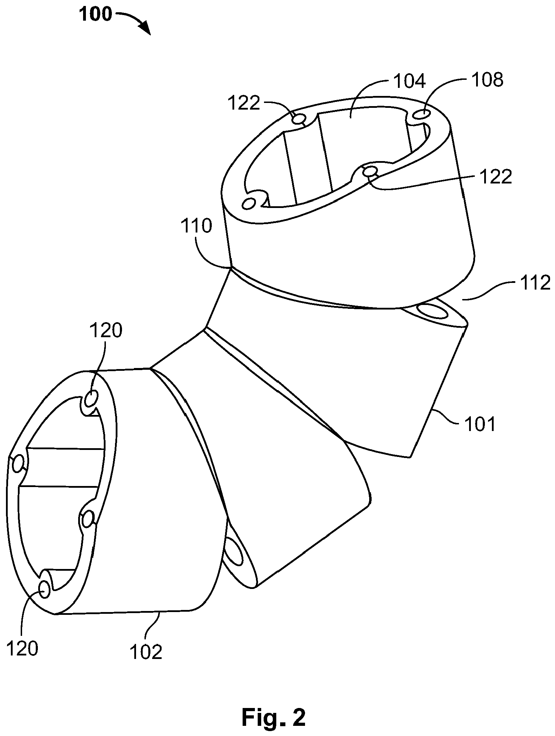

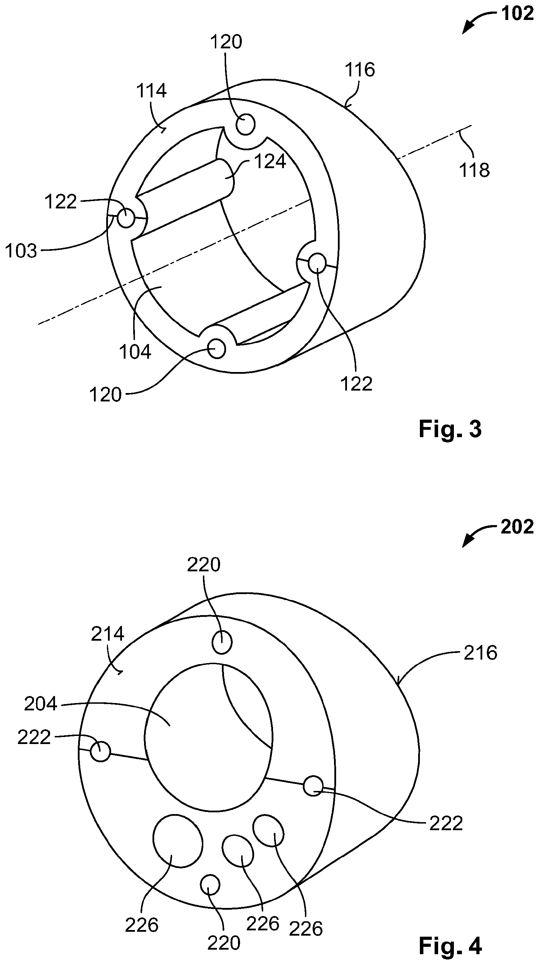

Fig. 1 shows a schematic perspective representation of an articulating shaft 100 according to a first example of the present invention. Fig. 2 shows a part of the articulating shaft 100 in a bent state. Fig. 3 is a perspective view of one of a plurality of segments 102 being part of the shaft 100. It should be noted that in the drawings the steering wires and other elements that are inserted into the various lumens are not shown in Fig. 2 and 3.

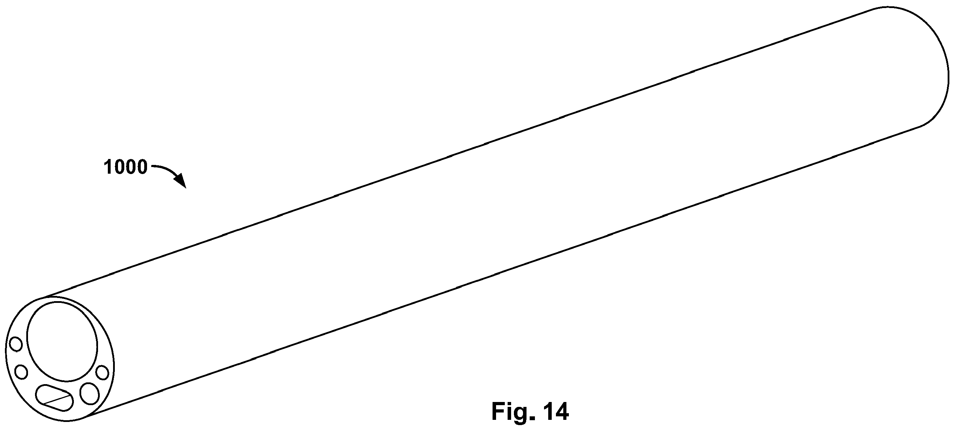

According to the present disclosure, a tubular body 101 of the articulating shaft 100 is fabricated from one single durometer extrusion part, having all lumens that are required. Such an extrusion part is referred to a multilumen extrusion (MLE). Figure 14 shows an example of an MLE 1000 with six lumens. The single durometer extrusion can be cut in various ways to modify its flexibility along the length as required, from a relatively stiff and pushable proximal section with non to minimal cutting, to a highly flexible distal steerable section with wide openings.

The extrusion can be made with multiple lumens to guide and position the necessary pull wires required for articulation, axial support wires to provide increased axial support and to reinforce or provide hinges for the bending of the sections. Additional lumens can be easily provided in the manufacture of the MLE to guide and position other elements needed in the construction of the devices using such a construction, for instance leads, fiber optic cables, working channels etc. This construction results in a steerable sheath composed of a minimal number of components.

According to the example shown in Fig. 1, a main lumen 104 extends along the complete length of the shaft 100. According to the example shown in Fig. 1, the articulating shaft 100 comprises steering wire support elements 106 with feed-through openings 108. The feed-through openings 108 are linearly arranged in a longitudinal direction of the shaft 100, so that segmented lumens 120 for a steering wire 128 is formed. Due to the particular shape of the segments 102, first removed sections or cuts 110 are formed between adjacent segments 102. These removed sections or cuts 110 facilitate the bending of the articulating shaft 100, if a steering wire is inserted into the row of feed-through openings 108 and exerts bending forces on the shaft 100. The presence of the second removed sections or cuts 112 arranged opposite to the first removed sections or cuts 110 allows the outer surface of the shaft 100 to elongate to facilitate articulation.

As can be seen from Fig. 3, each segment 102 has an essentially cylindrical shape with a first and a second lateral face 114, 116 delimiting the segment 102 in an axial direction, wherein the lateral faces are at partly inclined to the longitudinal axis at an angle different from 90°. In Fig. 3, the lower halves of the first and the second lateral face 114, 116, respectively, are orthogonal to the longitudinal axis, while the upper halves of the lateral faces 114, 116 are forming an angle deviating from 90° by about 20°. As is apparent from the depiction of an unbent shaft 100 shown in Fig. 1, these slanted parts of the lateral faces form wedge shaped removed sections or cuts 110 between the adjacent segments 102.

The segments 102 comprise four wire support elements, which are equally distanced around the circumference of the shaft 100 around the longitudinal axis 118. The feed-through openings are aligned along the longitudinal axis 118 to form four lumens, two of them being steering wire lumens 120. The steering wire lumens 120 contain the steering wires 128, 129 used for articulation.

The shaft 100 according to the first example further comprises reinforcement lumens 122 for receiving the axial support wires. These axial support wires may for instance be made from steel, Nitinol, or other suitable materials having some elastic properties. The axial support wires may also be referred to as the neutral axis support wires because they stabilize the segments 102 formed by the removed sections or cuts 110, 112 in their alignment and position. When a tensile load is applied, the actuating wire shortens and causes the openings 110 to close, thereby causing the assembly to bend in the direction of the openings 110. The neutral axis support wires 130 arranged in the reinforcement lumens 122 also bend in order to allow the assembly to move. The reinforcement wires also carry some of the articulation axial load and keep tension on the assembly, thereby preventing the segments from separating. Advantageously, the neutral axis support wires 130 are arranged at 90° spacing to the pull wires 128, 129.

In the shown embodiment, two axial support wire lumens 122 are provided. It is clear for a person skilled in the art, however, that any other number of lumens may also be provided. Further, also additional lumens for electrical or optical cables or fluid channels can be provided.

In the shown example, the shaft 100 has an essentially circular outer contour. However, any other cross-sectional outline may also be chosen, for instance an oval or polygonal contour. As shown here, also all openings forming a lumen have a circular cross-section. This is not necessarily the case; any other suitable cross-section, e. g. oval or polygonal, may also be used.

According to an advantageous aspect of the present invention, the main lumen 104 takes up most of the cross-sectional area of the shaft 100. In particular, the walls 124 delimiting the steering wire lumens 120 and the reinforcement lumens 122 bulge into the main lumen 104.

Fig. 1 to 3 show a tubular body fabricated from an MLE with one single internal working lumen 104. If additional lumens, e. g. for electric cabling are needed, the main lumen 204 may also be arranged off-center, as is shown in Fig. 4. Here, the outer contour of the segment 202 is identical to the outer contour of the segment 202 shown in Fig. 3. However, the main lumen 204 is arranged closer to one of the steering wire lumens 220, leaving room for further lumens 226. The additional lumens are provided for electrical leads, fiber optic cables etc. They can be arranged as required by the required device functions.

Reverting to Fig. 1 to 3, as mentioned above, the tubular body 101 is fabricated as one integral MLE, wherein the distal end of the extrusion has openings 110, 112 made in a direction normal to the axis of the extrusion. These openings (also referred to as removed sections or cuts) are made in such a way as to allow the distal end to bend in the direction of the openings. These openings effectively divide the extrusion into small sections, each joined to its adjacent segment with a thin strip of material which effectively works as a hinge. However, the segments 102 may also be separated from each other completely.

A pull wire is fitted into the pull wire lumen and fixed at the distal end segment. When this pull wire is pulled through the lumen at the proximal end it reduces the length of wire in the lumen and this has the effect of closing the openings. This causes the distal end to bend into a minimum radius curve defined by the opening size.

As indicated schematically in Fig. 1, the individual segments 102 are aligned along the longitudinal axis 118 of the shaft 100 and kept in position by the steering wires 128, 129 and the neutral axis support wires 130. The wedge-shaped first removed sections or cuts 110 facilitate bending of the shaft 100 when the steering wire 128 is pulled and thereby shortened. On the other hand, the narrow removed sections or cuts 112, which are formed by the abutting individual segments 102, open as shown in Fig. 1 and reduce the mechanical resistance against bending. The walls 124 bulge into the main lumen 104, thereby reducing the overall material thickness of the cross section of the shaft 100. This allows for a particularly light weight shaft and a maximized cross sectional area of the main lumen 104.

For fabricating the articulating shaft 100, the multi lumen extrusion forming the tubular body 101 is provided with openings 110 made on one side approximately 170° circumferentially, terminating by an equal amount from the neutral axis, thereby leaving a small amount of uncut material connecting each segment and forming a polymer hinge between the segments. This polymer hinge is not visible in the drawings. Further cuts 112 are made on the other side aligning in an axial direction with the centers of the openings 110 on the first side. These cuts are also made approximately 170° circumferentially, terminating by an equal amount from the neutral axis and forming the other side of the polymer hinges. The openings 110 are shaped in such a way as to allow the sheath to curve in a defined radius when the pull wire is shortened relative to the sheath, as can be seen in Figure 2.

The cuts 112 on the radially opposite side of the openings 110 allow the extrusion 101 to open on that side and allowing the outer surface of the MLE to effectively elongate to enable the bend to be made. The remaining material between the ends of the cuts 112 and the ends of the openings 110 is effectively the hinge 103 around which the segments 102 bend.

A pull wire 128 is inserted into the pull wire lumen 120. As the hinges 103 are necessarily of small width to allow articulation, neutral axis support wire lumens 122 can be provided. Flexible neutral axis support wires 130 are fitted into these lumens and fixed distally and proximally of the flexible section. These wires 130 can be made from stainless steel, Nitinol, or another flexible and axially stiff material. These wires 130 reinforce the flexible joints and provide high axial stiffness to the assembly to reduce axial compression under pull wire loads fed into the distal end. This improves articulation accuracy and precision. Furthermore, the neutral axis support wires 130 provide high column strength to the polymer hinges 103 to prevent possible buckling under axial loads. The wires 130 also provide elasticity to the system to provide springback of the distal tip during articulation.

Furthermore, the wires 130 can also provide a connection between the segments 102 so that the construction can be achieved using segments 102 with minimal polymer hinges 103 or even can be achieved with discrete separate segments 102 assembled on to the wires 130.

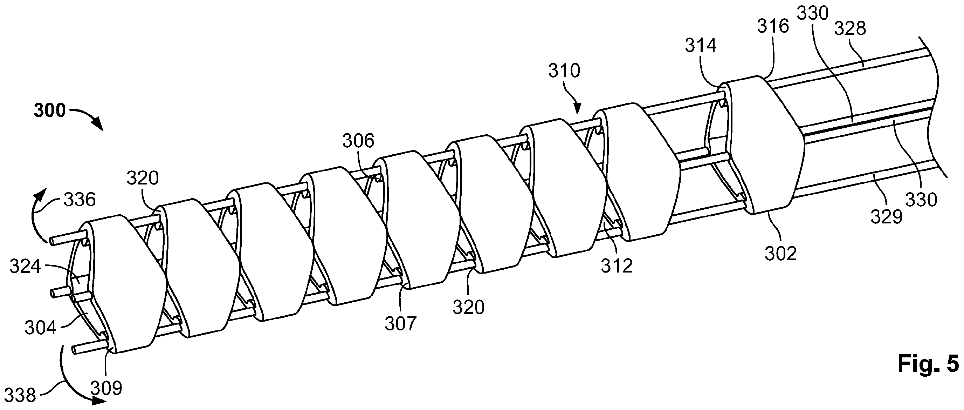

Fig. 5 illustrates a two-way steerable configuration of a shaft 300 with aligned openings 310, 312 and two pull wires. To this end, the first removed sections or cuts (or openings) 310 and the second removed sections or cuts 312 are symmetric with respect to a diameter interconnecting the neutral axis support wires 330. Consequently, a first steering wire 328 is fed through the steering wire lumen 320 and causes, when actuated, a bending of the shaft 300 in the direction shown by arrow 336. Furthermore, the second steering wire 329 is held in the feed-through openings forming the second steering wire lumen 320. Actuating the second steering wire 329 causes the shaft 300 to bend in the direction 338. The shaft 300 according to this example has the advantage that it is light weight, easy to fabricate and has high pliability in two directions Thus, accurate steering of the catheter system can be achieved with a minimum of components, in this case an MLE, two pull wires and two axial support wires.

The segments 302 may be separate components with no connections to adjacent segments.

The reinforcement or axial support wires 330 may for instance be made from steel, Nitinol, or other suitable materials having some elastic properties. The axial support wires 330 may also be referred to as the neutral axis support wires because they stabilize the segments formed by the removed sections or cuts 310, 312 in their alignment and position and are located on the neutral axis of the shaft which is the axis about which the shaft bends and the length of this axis remains constant during articulation of the shaft. For efficient articulation, the neutral axis length must not reduce or compress under the loads applied to the shaft by the steering wires. The neutral axis support wires provide this axial stiffness where the steering loads are higher than the ability of the polymer hinges to resist compression and buckling under this load. When a tensile load is applied, the actuating wire 328, 329 on the respective side shortens and causes the openings 310, 312 on the side of the actuated wire to close, thereby causing the assembly to bend in the direction of those openings 310, 312. The neutral axis support wires 330 arranged in the axial support wire lumens can also bend in order to allow the assembly to move. The axial support wires 330 also prevent the separate segments 302 from moving apart and reinforce the tensile strength of the polymer hinges for non separate segments to prevent the polymer hinges from breaking under tensile loads.

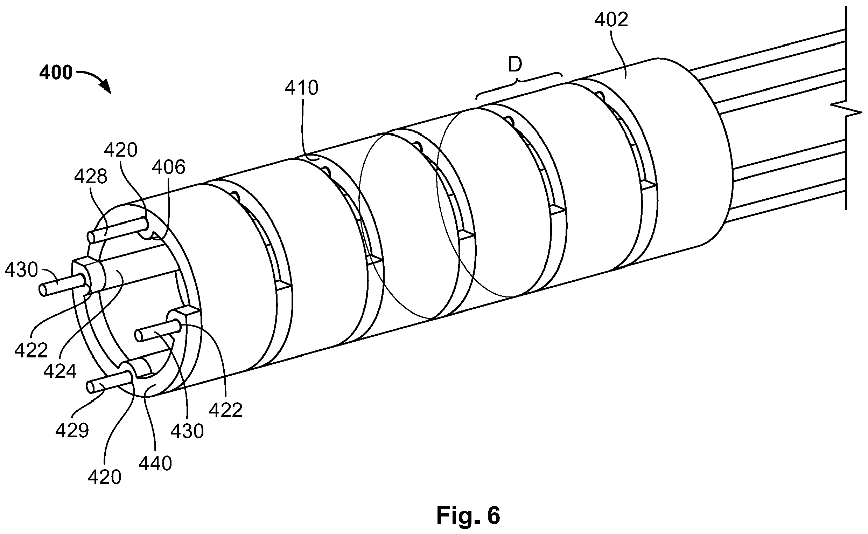

Fig. 6 illustrates an example of a one way steerable shaft 400. For this example, the MLE may be cut into segments with cuts essentially perpendicular to the longitudinal MLE axis. Spacers can be assembled on to the axial support wires and between each of the square cut segments. These spacers provide clearance on one or both sides of the assembly to allow the assembly to bend to one or both sides. The spacers can be of cylindrical form. They may be made from thin slices or sections 440 cut from the MLE with material removed to allow bending in one or both directions.

According to this example, each of the separate segments 402 have a uniform radial dimension D around their circumference. In order to enhance pliability, the segments 402 are separated from each other by spacer elements 440 (which may also be referred to as hinges). The spacer elements 440 extend around slightly more than 180° of the shaft's cross section. Thus, the spacer elements 440 form a part of the axial support wire lumens 430 as well as of the steering wire lumen 420, at the same time leaving open removed sections or cuts 410 between adjacent segments 402. The spacer elements 440 may either be loosely attached to the segments 402 allowing the segments to separate from the spacer elements upon actuation or may be firmly attached to an adjacent segment on one side. The proximal faces of the spacer elements 440 may for instance be glued or welded to the distal faces of the segments 402, each individual assembly of 440 and 402 forming a combined segment.

If a two-way steering shaft is desired, the spacers 440 may also be arranged on the upper half and the lower half of the shaft 400 in an alternating manner (not shown in the Figure).

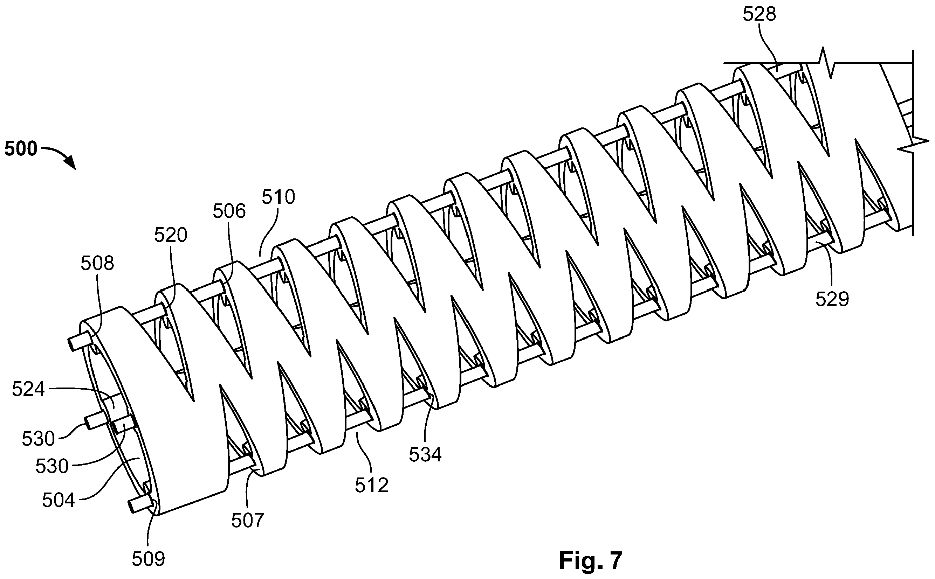

Fig. 7 illustrates a variation of an articulating shaft 500, where the MLE is provided with radially opposed openings that are not aligned in an axial direction. As shown in Fig. 7, the shaft 500 has a main lumen 504 which extends along the complete length of the shaft 500. According to the example shown in Fig. 7, the articulating shaft 500 comprises first wire support elements 506 with first feed-through openings 508 and second wire support elements 507 with second feed-through openings 509. The feed-through openings 508, 509 are linearly arranged in the longitudinal direction of the shaft 500, so that two segmented lumens 520 for two steering wires 528, 529 are formed. Thus, a two way steerable catheter can be provided using the shaft 500 shown in Fig. 7.

The shaft 500 may be formed as a MLE part with removed sections or cuts 510, 512 cut into the material by means of laser machining or sawing. Similar to the design shown in Fig. 5, the shaft 500 shown in Fig. 7 has removed sections or cuts 512 which are arranged radially opposing to the removed sections or cuts 510, thus allowing a two-way steering of the shaft 500. In contrast to the segmented arrangement of Fig. 5, the second removed sections or cuts 512 are staggered in an axial direction with respect to the first removed sections or cuts 510. This generates a zigzag structure of the outer wall of the shaft 500, which thus has a high pliability, at the same time providing a stable support at the neutral axis. Neutral axis support wires 530 further enhance the stability and increase the shaft torque capability.

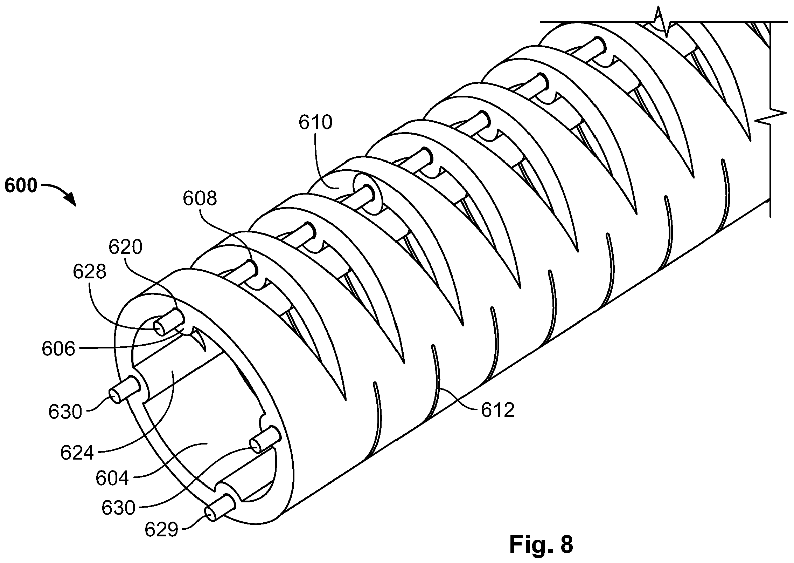

Fig. 8 shows a one-way steerable shaft 600 which is fabricated as one integral piece from an MLE. A main lumen 604 extends along the complete length of the shaft 600. The articulating shaft 600 comprises first wire support elements 606 with first feed-through openings 608. The feed-through openings 608 are linearly arranged in the longitudinal direction of the shaft 600, so that a segmented lumen 620 for the steering wire 628 is formed. Corresponding to the design shown in Fig. 1, the second removed sections or cuts 612 are narrow and not wedge shaped like the first removed sections or cuts 610. The second removed sections or cuts 612 thereby only allow opening of the shaft 600 when actuating the steering wire 628. In the shown example, the removed sections or cuts 610, 612 partly intersect the axial support wire lumens 622. However, the walls of the axial support wire lumens 622 may of course also be continuously closed along the longitudinal axis of the shaft 600.

When the shaft 600 is bent in a direction toward the feed-through openings 608, the openings 610 close, but also the second removed sections or cuts 612 open, thereby facilitating the bending process.

In contrast to the arrangement of Fig. 1, the second removed sections or cuts 612 are not aligned axially with the first removed sections or cuts 610, but are off-set in the axial direction for providing an enhanced mechanical stability of the shaft 600.

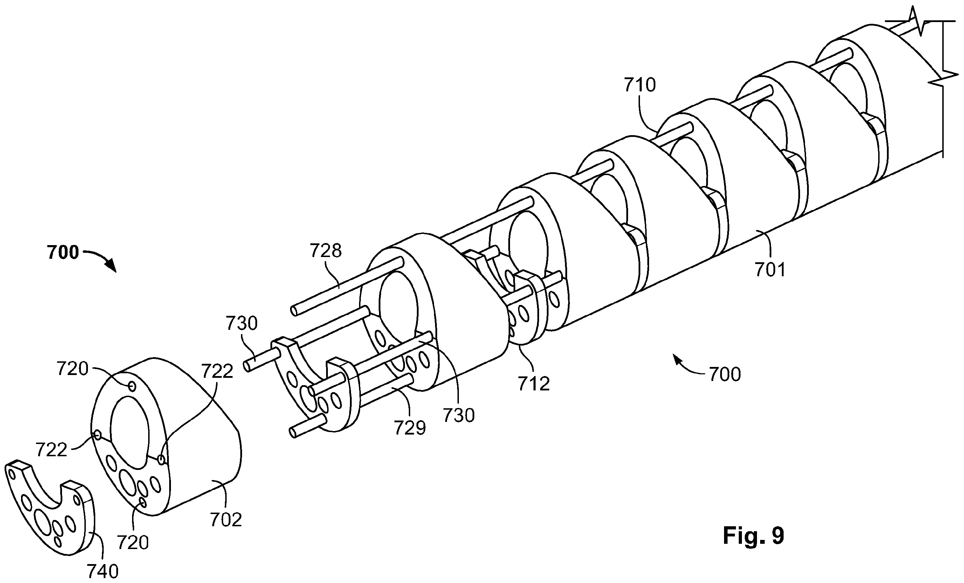

Further, as already mentioned above, also additional lumens for electrical cables or fluid channels can be provided. An example for such a shaft 700 is illustrated in Fig. 9.

For this example, the MLE may be cut into segments with wedge shaped first cuts 710 that are slanted at about half of the radius, and second cuts 712, which are essentially perpendicular to the longitudinal MLE axis. Spacers 740 can be assembled on to the axial support wires and between each of the square cut parts of the segments 702. These spacers 740 provide clearance on one or both sides of the assembly to allow the assembly to bend to one or both sides. The spacers can be of cylindrical form. They may be made from thin slices or sections cut from the MLE with material removed to allow bending in one or both directions.

According to this example, each of the separate segments 702 are formed as shown in Fig. 4 and are separated from each other by spacer elements 740 (which may also be referred to as hinges). The spacer elements 740 extend around slightly more than 180° of the shaft's cross section. Thus, the spacer elements 740 form a part of the axial support wire lumens 730 as well as of the steering wire lumen 720, at the same time leaving open removed sections or cuts 710 between adjacent segments 702. The spacer elements 740 may either be loosely attached to the segments 702 allowing the segments to separate from the spacer elements 740 upon actuation or may be firmly attached to the segments 702. The spacer elements 740 may for instance be glued or welded to an adjacent segment on one side. The proximal faces of the spacer elements 740 may for instance be glued or welded to the distal faces of the segments 702, each individual assembly of 740 and 702 forming a combined segment.

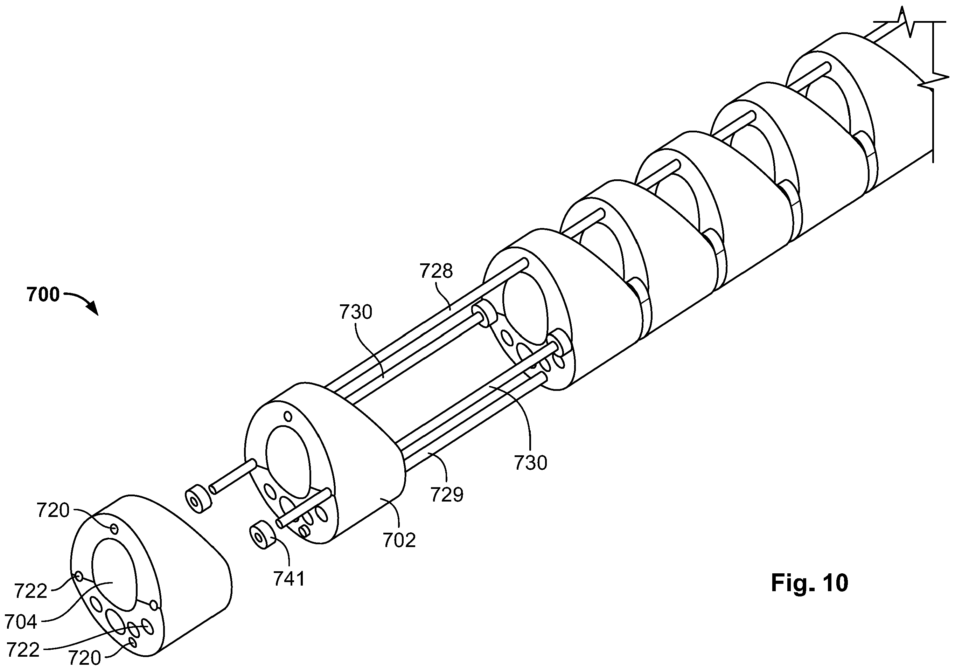

An alternative example of the actuating shaft 700 is shown in Figure 10. Instead of using the relatively large spacers 740 fabricated for instance from the MLE, the segments 702 of the shaft 700 may also by distanced from each other by simple cylindrical spacers 741, which only surround the neutral support wires 730.

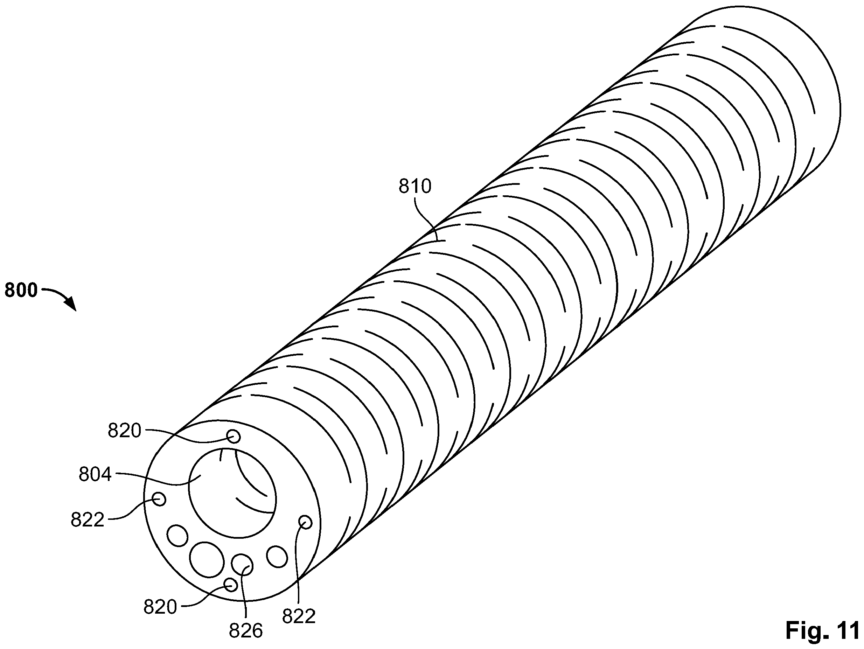

According to the present disclosure, the articulating shaft does not necessarily have to be shaped identical along its complete longitudinal extension. For instance, the geometries explained with reference to Figures 1 to 10 may only be used for the distal region of the shaft, where a high flexibility is needed. The proximal section of the shaft may be shaped differently. For instance, as shown in Fig. 11, a proximal section of the shaft 800 can be fabricated from a MLE or fabricated from the same MLE as the distal articulating section and can be provided with a plurality of incisions 810 to increase the shaft's flexibility. The size, geometry, and pitch of the incisions 810 may vary along the longitudinal length in order to modify the flexibility of the sheath 800 along its length.

The articulating shaft may be constructed from a single MLE with distal cut pattern arrangements as previously outlined to allow articulation in one or more directions of the distal section and different cut patterns designed to give varying flexibilities from distal section to proximal end or no further modifications to the MLE for the mid and proximal sections to result in a complete distal to proximal shaft constructed with a minimum of simple and low cost components, a single MLE, one to two pull wires and one to two axial support wires.

In all the shown examples, the shaft has an essentially circular outer contour. However, any other cross-sectional outline may also be chosen, for instance an oval or polygonal contour. As shown here, also all openings forming a lumen have a circular cross-section. This is not necessarily the case; any other suitable cross-section, e. g. oval or polygonal, may also be used.

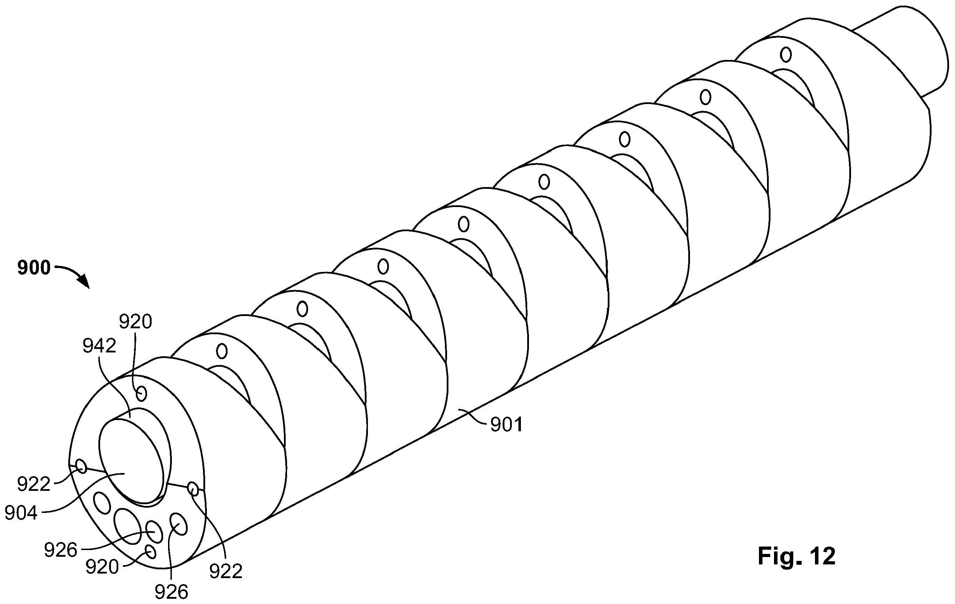

Furthermore, in order to create one ore more fluid tight channels, at least one of the lumens may be lined with a tube e. g. fabricated from a polymer. Fig. 12 illustrates a shaft 900 wherein a polymer tube 942 is inserted into the main lumen 904. Of course, alternatively or additionally, also one or more of the additional lumens 926 may be provided with a fluid-tight liner 942.

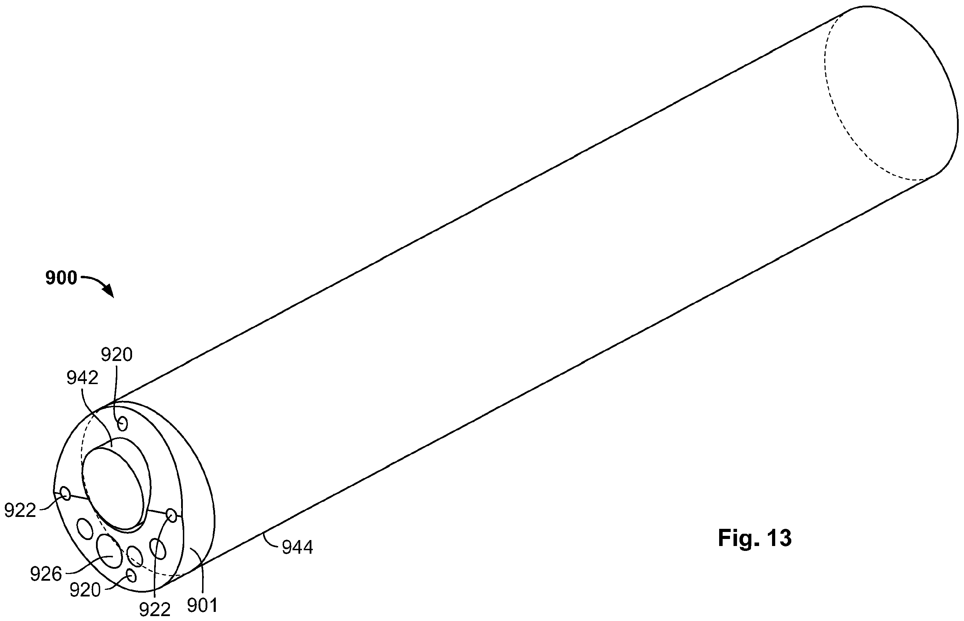

Additionally or alternatively, as shown in Fig. 13, a polymer jacket 944 may be fitted over the outer surface of the multi lumen extrusion in order to provide a smooth surface for contact with anatomy and to protect the other components of the completed device from external substances, e. g. bodily fluids. The jacket 944 may comprise a simple extruded polymer tube, a braid or coil reinforced tube, or any other suitable construction.

In summary, the present invention provides an articulating shaft design based on a multi lumen extrusion. A multi lumen extrusion may for instance be made from a hard polymer material or other suitable materials. This extrusion part may have articulation lumens arranged 180° apart and close to the outer surface. Wires or cables can be fed through these lumens. Removed sections or cuts are arranged to cross these articulation lumens.

The extrusion may have neutral axis support lumens arranged close to the surface and at 180° to each other. These lumens may advantageously be arranged at 90° to the articulation lumens.

Axial support wires may be assembled into these lumens. These can be steel or Nitinol or other suitable materials with some elastic properties. These axial support wires can also be referred to as the neutral axis support wires. Articulation wires are assembled into the articulation lumens. When these wires are attached to the distal end of the assembly and a tensile load applied, then the wire (or cable) shortens. This causes the openings to close, causing the assembly to bend in the direction of the openings. The neutral axis support wires bend to allow the assembly to bend. These axial support wires also carry some articulation axial load. The wires keep the segments in alignment and position. The wires also keep tension on the assembly, preventing the segments from separating.

The segments can be configured in a multitude of ways, with single-way and two-way steering possible, with aligned openings (leading to separate segments) or alternating segments giving a single piece design.

Multiple lumens can be included to carry various elements.

The openings can be mechanically cut or laser cut, by means of a gang saw, grinder, blades, or other methods.

Individual segments can be cut with square faces and hinge pieces assembled between the square segments.

For one-way steering, the redundant steering lumen can be used for an additional support wire to enhance torque performance. This wire is fixed at the distal end only and is free to move axially as the part is articulated.

**REFERENCE NUMERALS**

| **Reference Numeral** | **Description** |
|---|---|
| 100, 200, 300, 400, 500, 600, 700, 800, 900 | Articulating shaft |
| 101, 701, 901 | Tubular body |
| 102, 202, 302, 402, 702 | Segment |
| 103 | Hinge |
| 104, 204, 304, 404, 504, 604, 704, 804, 904 | Main lumen |
| 106, 206, 306, 406, 506, 606 | Wire support elements |
| 307, 507 | Second wire support elements |
| 108, 508 | Feed-through opening |
| 509 | Second feed-through opening |
| 110, 210, 310, 410, 510, 610, 710, 810 | First removed sections or cuts |
| 112, 212, 312, 512, 612, 712 | Second removed sections or cuts |
| 114, 214, 314 | First lateral face |
| 116, 216, 316 | Second lateral face |
| 118 | Longitudinal axis |
| 120, 220, 320, 420, 520, 620, 720, 820, 920 | Steering wire lumen |
| 122, 222, 322, 422, 522, 622, 722, 822, 922 | Reinforcement lumen |
| 124, 324, 424, 524, 624 | Wall bulging into main lumen |
| 226, 826, 926 | Additional lumen |
| 128, 328, 428, 528, 628, 728 | (First) steering wire |
| 129, 329, 529, 729 | Second steering wire |
| 130, 330, 430, 530, 630, 730 | Neutral axis support wire |
| 336 | First bending direction |
| 338 | Second bending direction |
| 440, 740 | Spacer element |
| 741 | Cylindrical spacer element |
| 942 | Polymer tube |
| 944 | Outer jacket |
| 1000 | Multi lumen extrusion (MLE) |

## Claims

1. Articulating shaft for a steerable catheter system, the shaft (100) comprising:
a tubular body (101) with a longitudinal central axis (118), the tubular body having at least one main lumen (104) with a distal end and a proximal end, and
at least one wire support element (106) forming at least one wire lumen (108) for supporting at least one actuating wire (128),
wherein the main lumen (104) and the wire lumen (108) are integrally formed in an extrusion part having a uniform hardness in an axial and a radial direction,
wherein the shaft (100) further comprises a plurality of removed sections or cuts (110) which extend in a radial direction and are distanced from each other in an axial direction.

2. Articulating shaft according to claim 1, further comprising a flexible outer jacket (944) encompassing the tubular body (901).

3. Articulating shaft according to claim 1 or 2, wherein the at least one main lumen (904) comprises a flexible tube (942) inserted into the tubular body (901).

4. Articulating shaft according to one of the preceding claims, wherein the tubular body (101) comprises a plurality of segments (102), each of the segments being at least partly separated from each other in an axial direction by one of the plurality of removed sections or cuts (110), the removed sections or cuts (110) intersecting an inner wall of the main lumen (104) and wherein each of the removed sections or cuts (110) extend by at least 180° in a radial direction around the body (101).

5. Articulating shaft according to one of the preceding claims, wherein the shaft (100) comprises a plurality of discrete segments (102) aligned along the longitudinal central axis (118).

6. Articulating shaft according to claim 5, wherein the discrete segments (402) are aligned with at least one separate spacer element (440) between each pair of adjacent segments (402).

7. Articulating shaft according to claim 6, wherein the segments (402) each have the shape of a straight circular cylinder and the spacer elements (440) have an essentially C-shaped radial outline, leaving axially distanced removed sections or cuts (410) between the segments.

8. Articulating shaft according to one of the claims 5 to 7, wherein each of the segments (102) has a first and a second lateral face (114, 116) delimiting the segment (102) in an axial direction, wherein at least one of the lateral faces (114, 116) is at least partly inclined to the longitudinal axis (118) at an angle different from 90°.

9. Articulating shaft according to one of the preceding claims, wherein the shaft (100) has a circular radial outline and the removed sections or cuts (110) have a wedge-shaped tapered outline, wherein the wire support elements (106) are located in a region where the removed sections or cuts (110) have their minimum axial dimension.

10. Articulating shaft according to one of the preceding claims, wherein a plurality of first removed sections or cuts (110) is provided separating a plurality of first wire support elements (106) from each other, and wherein a plurality of second removed sections or cuts (112) is provided opposing the first removed sections or cuts (110) in a radial direction and being arranged off-set from the plurality of first removed sections or cuts (110) in an axial direction.

11. Articulating shaft according to one of the preceding claims, wherein the main lumen (104) is arranged concentrically within the outline of the shaft (100).

12. Articulating shaft according to one of the claims 1 to 10, wherein the main lumen (204) is arranged within the outline of the shaft (200) with its central axis being distanced from the longitudinal central axis of the shaft (200).

13. Articulating shaft according to one of the preceding claims, wherein the at least one wire support element (106) comprises a tube shaped wire lumen with an inner wall and an outer wall, the outer wall being partly formed by an inner wall of the main lumen.

14. Articulating shaft according to one of the preceding claims, wherein the tubular body (101) is fabricated from a single multilumen extrusion, MLE.

15. Steerable wire guiding catheter system comprising an articulating shaft (100) according to one of the preceding claims,
at least one actuating wire (128) passing through the at least one wire lumen, wherein the actuating wire (128) has a proximal end and a distal end, the actuating wire being fixed so that pulling the actuating wire effects steering of the catheter, and
control means located at a proximal portion of the catheter for controlling the pulling on the proximal end of the actuating wire (128).

16. Method for fabricating an articulating shaft (100) for a steerable catheter system, the method comprising:
providing a tubular body (101) with a longitudinal central axis (118), the tubular body (101) having at least one main lumen (104) with a distal opening and a proximal opening, and at least one wire support element (106) forming at least one wire lumen (108) for supporting at least one actuating wire (128),
wherein the main lumen (104) and the wire lumen (108) are integrally formed in an extrusion part having a uniform hardness in an axial and a radial direction,
providing a plurality of removed sections or cuts (110) which extend in a radial direction and are distanced from each other in an axial direction.

17. Method according to claim 16, wherein the tubular body (101) is fabricated from a single multilumen extrusion, MLE.
